# EUROPEAN PATENT APPLICATION

(11) **EP 4 792 899 A1**
(43) Date of publication of application: **19.08.2026**
(21) Application number: 24877081.0
(22) Date of filing: 02.10.2024
(51) Int. Cl.: A63B 69/00, G06Q 50/10, G16H 40/00

(54) **INFORMATION PROCESSING DEVICE, INFORMATION PROCESSING DEVICE CONTROL METHOD, AND INFORMATION PROCESSING DEVICE CONTROL PROGRAM**

(30) Priority: 10.10.2023 JP 2023175282
(71) Applicant: ASICS Corporation, Kobe-shi, Hyogo 650-0021 (JP)
(72) Inventor: MIYAZAKI Yosuke, Kobe-shi, Hyogo 650-0021 (JP); INABA Ryuichiro, Kobe-shi, Hyogo 650-0021 (JP)
(74) Representative: TBK
(86) International application number: PCT/JP2024/035315
(87) International publication number: WO 2025/079483

(57) **Abstract**

An information processing device, for example, related to a training support system that updates the training program in accordance with the daily training status of the user and maintains the motivation of the user. An information processing device that provides a user with a training program related to running according to one embodiment of the present invention includes: a setting unit configured to set, as the training program, a training menu to be implemented in a predetermined period and a day of implementation of the training menu; an acquisition unit configured to acquire implementation information regarding whether the training menu set for the day of implementation has been implemented by the user; a determination unit configured to determine whether the training menu for a current day of the day of implementation has been implemented; an update unit configured to update, on the basis of predetermined conditions, training menus for days following the current day of the day of implementation if the determination unit determines that the training menu for the current day of the day of implementation has not been implemented; and a display processing unit configured to cause a terminal to display the updated training menus.

## Description

### BACKGROUND

### Technical Field

This invention relates to an information processing device, an information processing device control method, and an information processing device control program.

### Background Information

A service for promoting continuation of an exercise habit of a user has been provided conventionally. For example, patent document 1 discloses collecting exercise information of a user and providing direct, visual feedback to motivate a user to maintain a regular exercise program.

### Prior Art Documents

### Patent Documents

Patent Document 1: Japanese Unexamined Patent Application Publication No. 2017-073125

### SUMMARY

### Problems to be Solved by the Invention

Even if exercise training programs for running, walking, and cycling, for example, are provided, situations may arise on a daily basis where execution of the programs is hindered by bad weather, poor physical condition, or lack of available time caused by work. Even on days when the training menu is in line with the training program, the user may be unable to perform training as per the menu provided depending on the physical condition of the user and availability of time. This lack of programmatic training can lead to a loss of motivation of the user.

Accordingly, a training support system has been needed in which training programs are updated to an appropriate state for a user in accordance with daily training conditions of the user, thereby maintaining the motivation of the user.

### Means to Solve the Problems

An information processing device that provides a user with a training program related to running according to one embodiment of the present invention includes: a setting unit configured to set, as the training program, a training menu to be implemented in a predetermined period and a day of implementation of the training menu; an acquisition unit configured to acquire implementation information regarding whether the training menu set for the day of implementation has been implemented by the user; a determination unit configured to determine whether the training menu for a current day of the day of implementation has been implemented; an update unit configured to update, on the basis of predetermined conditions, training menus for days following the current day of the day of implementation if the determination unit determines that the training menu for the current day of the day of implementation has not been implemented; and a display processing unit configured to cause a terminal to display the updated training menus.

A method for controlling an information processing device that provides a user with a training program related to running according to one embodiment of the present invention causes the information processing device to perform the steps of: setting, as the training program, a training menu to be implemented in a predetermined period and a day of implementation of the training menu; acquiring implementation information regarding whether the training menu set for the day of implementation has been implemented by the user; determining whether the training menu for a current day of the day of implementation has been implemented; updating, on the basis of predetermined conditions, training menus for days following the current day of the day of implementation if it is determined that the training menu for the current day of the day of implementation has not been implemented; and causing a terminal to display the updated training menu.

A program for controlling an information processing device that provides a user with a training program related to running according to an embodiment of the present invention causes the information processing device to embody the functions of: setting, as the training program, a training menu to be implemented in a predetermined period and a day of implementation of the training menu; acquiring implementation information regarding whether the training menu set for the day of implementation has been implemented by the user; determining whether the training menu for a current day of the day of implementation has been implemented; updating, on the basis of predetermined conditions, training menus for days following the current day of the day of implementation if it is determined that the training menu for the current day of the day of implementation has not been implemented; and causing a terminal to display the updated training menu.

### Advantageous Effects of the Invention

An embodiment of the present invention provides an information processing device for a training support system that updates a training program to an appropriate state for a user in accordance with the daily training conditions of the user, thereby contributing to maintaining the motivation of the user.

### BRIEF DESCRIPTION OF THE DRAWINGS

Features, advantages, and technical and industrial significance of exemplary embodiments of the invention will be described below with reference to the accompanying drawings, in which like numerals denote like elements, and wherein:
FIG. 1 shows a schematic diagram illustrating a configuration of a training support system according to an embodiment of the present invention,
FIG. 2 shows an example of a functional block diagram illustrating a server (information processing device), a user terminal (communication terminal), and a sensor device according to an embodiment of the present invention,
FIG. 3 shows an example of a training program,
FIG. 4A shows an example of a first priority table according to an embodiment of the present invention, and FIG. 4B shows a schematic diagram illustrating an update process of the training program,
FIG. 5 shows a flowchart illustrating an example of an operation of the server according to an embodiment of the present invention,
FIG. 6 shows a flowchart illustrating an example of an operation of the server according to an embodiment of the present invention,
FIGS. 7A to 7C each show an example of a display screen of a user terminal according to an embodiment of the present invention,
FIGS. 8A to 8C each show a schematic diagram illustrating updating of a growth rate of running ability,
FIG. 9 shows an example of a second priority table according to an embodiment of the present invention,
FIGS. 10A and 10B each show a schematic diagram illustrating an update process of the training program according to an embodiment of the present invention, and
FIG. 11 shows an example of a display screen of a user terminal according to an embodiment of the present invention.

### DETAILED DESCRIPTION

With reference to the drawings, an embodiment of an invention according to the present disclosure (also referred to as the present invention) will be described. The drawings each are an example, and the invention is not limited to what is illustrated in the drawings. For example, the number of servers (information processing devices), user terminals (communication devices), database servers, and sensor devices and their size ratios, data sets (tables), display screens, training programs, and flowcharts shown in the drawings are examples, and the present invention is not limited to these.

### System Configuration

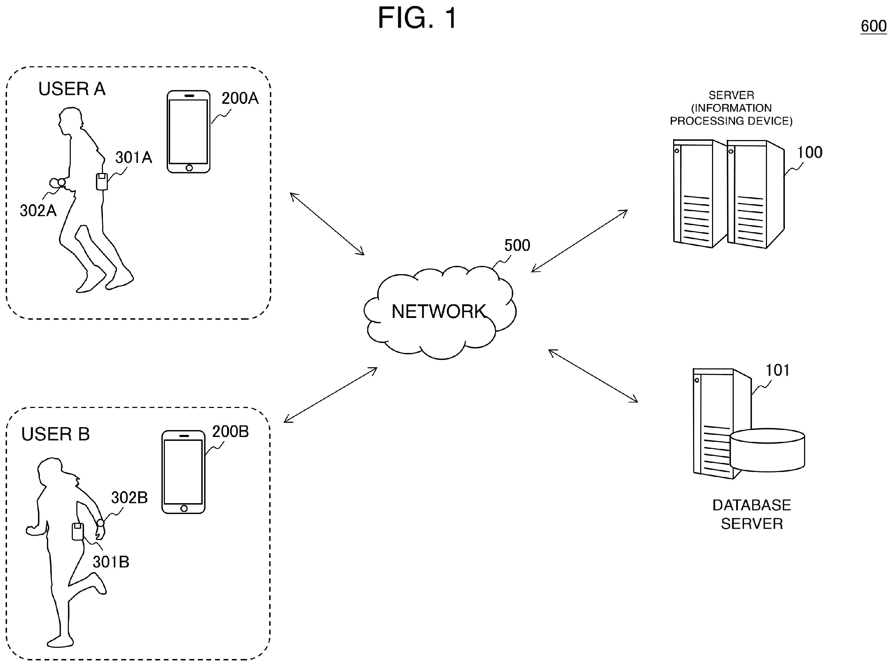

FIG. 1 shows a diagram illustrating an example of a configuration of a training support system according to an embodiment of the present invention. A training support system 600 may be directed to an information and communication system that supports training by a user (runner). According to one embodiment of the present invention, a training program may be set up for the user in accordance with the running ability of the user, and the training program may be adaptively updated in accordance with the implementation of the training by the user. Further, according to one embodiment of the present invention, information may be provided to the user regarding the rate of growth of the running ability in accordance with the implementation of the training program. The term "training" as used herein may include "practice," "discipline," and similar activities, and may include activities voluntarily performed by an individual as well as activities performed under guidance from a specialist or a trainer.

The training support system 600 may include at least a server 100, a database server 101, user communication terminals (user terminals) 200 (200A, 200B), and sensor devices 301 (301A, 301B), 302 (302A, 302B). In FIG. 1, two users A and B are shown, and the respective user terminals and sensor devices are marked with the alphabetic letters "A" and "B." However, the number of users may be greater than or equal to or less than or equal to this number, and user terminals and sensor devices may be provided in numbers corresponding to the users. Hereinafter, unless otherwise distinguished, user terminals and sensor devices will be referred to simply as user terminal 200 and sensor devices 301, 302, respectively. The sensor device 301 may be a motion sensor and the sensor device 302 may be a vital sensor as will be described in detail below.

The server 100 is capable of performing various processes related to the training support services embodied by the training support system 600. Further, the server 100 is connected to the user terminals 200 via the network 500. The network 500 may include a wireless network and/or a wired network such as, for example, a wireless local area network (WLAN), a wide area network (WAN), integrated services digital networks (ISDNs), wireless LANs, code division multiple access (CDMA), long term evolution (LTE), LTE-Advanced, fourth generation (4G) mobile communication systems, fifth generation (5G) mobile communication systems, sixth generation (6G) and later mobile communication systems, or any combination thereof.

The server 100 also transmits and receives various data to and from the database server 101. The database server 101 stores various types of data needed for embodying functions of the training support system 600. In FIG. 1, the server 100 and database server 101 are shown connected via the network 500, but the server 100 and database server 101 may be connected by a dedicated internal network. Although FIG. 1 shows the server 100 and the database server 101 separately, the database server 101 may function as the storage of the server 100, which will be described below. In FIG. 1, one server 100 and one database server 101 are shown respectively, but are not limited to this. That is, the functions described as provided by the server 100 may be embodied by multiple servers, or multiple database servers 101 may be provided. The server 100 may be, for example, a distributed server system that operates cooperatively by communicating over a network, or a cloud server. That is, the server 100 is not limited to physical servers, but may also include software virtual servers.

The user terminal 200 is directed to a communication terminal used by the user, and has applications installed to use the training support service (hereafter, also referred to as "training support application"). As described in detail below, the user terminal 200 may be able to transmit information acquired from the motion sensor device 301 and vital sensor 302 to the server 100 via the training support application.

Although FIG. 1 shows a smartphone as a user terminal 200, any terminal that is capable of embodying the functions described in the embodiments below may be used as a user terminal 200. For example, user terminal 200 may be directed to a computer (e.g., tablet, desktop computer, laptop), handheld computing device (by way of example, not limitation, wearable device (such as eyeglass-type device (smart glasses), watch-type device (smart watch)), and smart speakers, for example. The sensor device 302 may also function as the communication device 200 by providing the various functions of the communication device 200, which will be described below.

The user may wear the motion sensor device 301 and the vital sensor 302 to perform various training menus related to running. The "training menu" corresponds to a combination of an exercise menu that defines the type of running, such as jogging, interval running, and build-up running (which will be described below), and the distance traveled. The motion sensor device 301 and the vital sensor 302 correspond to wearable devices with communication functions that can be worn on the body of the user, acquire running information and biometric information of the user, and transmit the acquired information to the user terminal 200. The motion sensor device 301 is worn near the waist along the torso of the user and measures sensor data for computing the evaluation value of an index to evaluate the running form of the user as running information. Some types of motion sensor devices 301 may be attached to shoes. The vital sensor 302 also acquires biometric information of the running user. These running information and biometric information may be included in the "implementation information" described below, which relates to the determination of whether the user has implemented the training menu or not.

Although the motion sensor device 301 and the vital sensor 302 are shown separately in FIG. 1, the invention is not limited to this, and the motion sensor device 301 and the vital sensor 302 may be integrated into a single sensor device to acquire running information and biometric information, or multiple sensor devices may be used. The running information and biometric information will be described below.

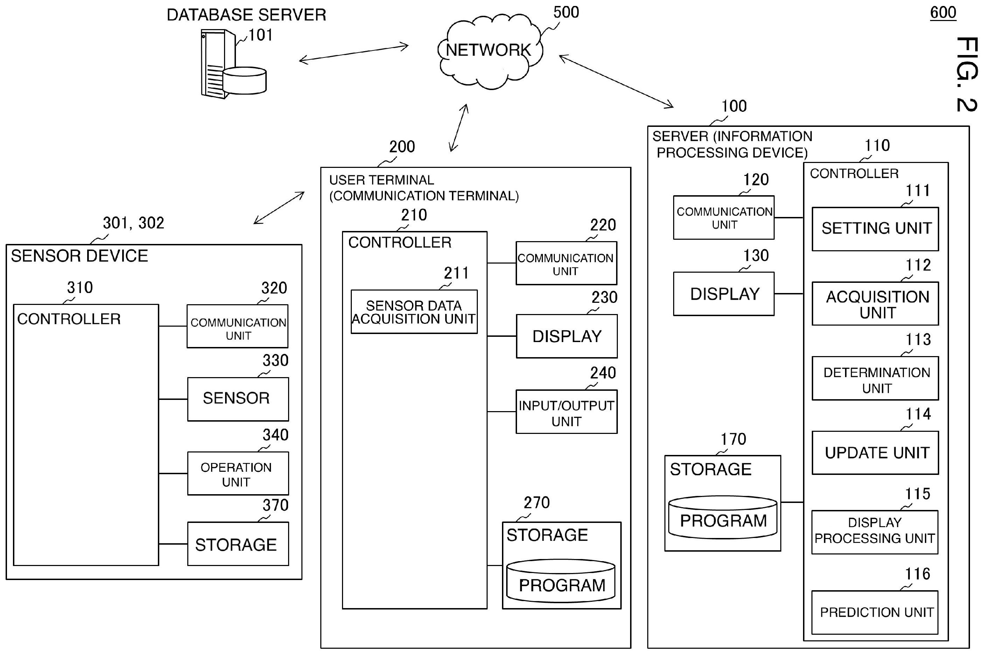

Next, with reference to FIG. 2, the hardware and functional configurations of the server 100, user terminal 200, motion sensor device 301, and vital sensor 302 will be described.

### Sensor Device

FIG. 2 shows an example of a block diagram illustrating a sensor device (motion sensor device 301, vital sensor 302). The motion sensor device 301 and the vital sensor 302 are described using the same block diagram because their basic configuration and functions are similar, with the only difference being the information to be acquired.

The motion sensor device 301 and the vital sensor 302 each include a controller 310, communication unit 320, sensor 330, operation unit 340, and storage 370. The controller 310 is configured by an arithmetic processor such as a CPU and acquires the output of the sensor 330 at predetermined time points by executing a program stored in the storage 370. The controller 310 also transmits the data acquired from the sensor 330 to the user terminal 200 via the communication unit 320.

In the motion sensor device 301, the sensor 330 detects, for example, acceleration occurring in a linear direction along three mutually perpendicular axes, angular velocity occurring in a rotational direction about three mutually perpendicular axes, or geomagnetism. The sensor 330 acquires sensor data such as acceleration and angular velocity at a time when the user runs as running information. The running information may be stored in the storage 370 along with information on the time when it is acquired.

In the vital sensor 302, the sensor 330 detects biometric information such as the body temperature, blood pressure, heart rate, and number of breaths per unit time of the user. The biometric information may be stored in the storage 370 along with information on the time when it is acquired.

The communication unit 320 serves to transmit and receive data to and from the user terminal 200 by wireless communication (e.g., Bluetooth (registered trademark) low energy: BLE) such as wireless local area network (LAN) and near field communication or by wired communication using a predetermined communication protocol. For example, the communication unit 320 transmits the running information and biometric information stored in the storage 370 to the user terminal 200. The communication unit 320 may collectively transmit the running information and biometric information stored in the storage 370 to the user terminal 200 after measurements by the motion sensor device 301 and the vital sensor 302 are completed, or may transmit the running information and biometric information stored in the storage 370 at predetermined time points during the measurement by the sensor 330.

The operation unit 340, for example, is configured by an input button or touch panel and accepts operations by the user. For example, in the motion sensor device 301 and the vital sensor 302, the measurement by the sensor 330 may be started, suspended, and stopped, for example, when an operation to start the measurement is entered by the user via the operation unit 340.

### User Terminal

### (1) Hardware Configuration of User Terminal

The user terminal 200 includes a controller 210, communication unit 220, display 230, input/output unit 240, and storage 270.

The controller 210 is typically directed to a processor embodied by a central processing unit (CPU), micro processing unit (MPU), and graphics processing unit (GPU), for example. The controller 210 reads the programs stored in the storage 270 and executes the codes or instructions contained in the programs to perform the functions and methods shown in the embodiments.

The controller 210 controls the communication unit 220, display 230, and input/output unit 240. Specifically, the controller 210 controls communication between the user terminal 200 and the server 100, motion sensor device 301, and vital sensor 302 by the communication unit 220, and transmits and receives various data between them. The controller 210 also controls the display of data on the display 230. For example, the controller 210 may cause the display 230 to display the training menu for the day of implementation regarding the training program set for the user as well as running ability predicted to be acquired by carrying out the training menu, for example. Further, the controller 210 controls the communication of various information to and from external devices via the input/output unit 240. For example, the controller 210 may transmit various types of information to the functional units in response to the input operations by the user accepted by the input device, or transmit information from the functional units to output devices not shown, such as touch panels, monitors, and speakers.

The storage 270 stores various programs and various data needed for the user terminal 200 to operate. For example, the storage 270 may store the programs for the training support application described above. The storage 270 may include, for example, flash memory, and may also include a memory (a random access memory (RAM), and read only memory (ROM), for example) that provides a work area for the controller 210.

The communication unit 220 is implemented as hardware such as a network adapter, communication software, and combinations thereof, and transmits and receives various data to and from the server 100 via the network 500. Such communication may be performed by either wired or wireless means, and any communication protocol may be used if they can communicate with each other. The communication unit 220 transmits various data to the server 100 in accordance with instructions from the controller 210. The communication unit 220 also receives various data transmitted from the server 100 and communicates them to the controller 210. The communication unit 220 also executes the wireless or wired communication described above between the communication unit 220 and the motion sensor device 301 and the vital sensor 302.

The display 230 may correspond to a monitor that displays data in accordance with the display data written into the frame buffer, such as a touch panel or touch display, for example.

The input/output unit 240 includes input devices for various operations on the user terminal 200 and output devices for outputting the processing results processed by the user terminal 200. The input devices may include, for example, touch panels, touch displays, cameras, and microphones, and the output devices may include, for example, displays, touch panels, and speakers.

### (2) Functional Configuration of User Terminal

The user terminal 200 includes a sensor data acquisition unit 211 as a function embodied by the controller 210.

The sensor data acquisition unit 211 acquires sensor data measured by the motion sensor device 301 worn by the user as running information. The sensor data acquisition unit 211 also acquires sensor data measured by the vital sensor 302 worn by the user as biometric information.

The running information is used to compute indicators related to the running form of the user. The indicators are elements indicating the quality of running of the user, and may include, for example, running speed, pitch, stride, a stride-to-height ratio, vertical oscillation, a vertical oscillation-to-height ratio, lateral movement, longitudinal movement, ground contact time, flight time, a ground contact time ratio, a flight time ratio, deceleration amount, sinking amount, a sinking amount-to-height ratio, sinking time, braking time, propulsion time, ground contact impact, push-off acceleration, push-off time, pelvic rotation amount, stiffness, a stiffness-to-body-weight ratio, a landing angle, a push-off angle, lateral impact, a posterior pelvic tilt amount, a pelvic drop amount, a pelvic lift amount, pelvic rotation time point after landing, a forward lean angle, and an impact peak slope. The indicators can characterize the running form of the user and can be used to estimate the fatigue level of the user.

Any known method may be used to compute the indicators. Known methods may include those described in Japanese Registered Patent Nos. 6648439, 6711433, 7155345, or 5849092, for example.

### Server

### (1) Hardware Configuration of Server

The server 100 includes a controller 110, communication unit 120, display 130, and storage 170.

The storage 170 is typically embodied by various storage media such as a hard disc drive (HDD), solid state drive (SSD), and flash memory, and serves to store various programs and data needed for the server 100 to operate. Further, the storage 170 includes a memory (e.g., RAM, ROM) that provides a work area for the controller 110.

The controller 110 is typically a processor, embodied by a central processing unit (CPU), MPU, and/or GPU, for example. The controller 110 reads the programs stored in the storage 170 and executes the codes or instructions contained in the programs to perform the functions and methods shown in the embodiments.

The communication unit 120 is implemented by hardware such as a network adapter, communication software, and combinations of these. The communication unit 120 may transmit and receive various data to and from the user terminal 200 via the network 500 using any communication protocol.

### (2) Functional Configuration of Server

The server 100 includes a setting unit 111, an acquisition unit 112, a determination unit 113, an update unit 114, a display processing unit 115, and a prediction unit 116 as functions embodied by the controller 110. In FIG. 2, the functional units that are not essential for the embodiments described hereinafter may be omitted. The function or processing of the functional units may be embodied by machine learning or AI to the extent feasible.

The setting unit 111 sets a training menu to be implemented during a predetermined period and a day of implementation of the training menu as a training program related to running. The various exercises that configure the training program are referred to here as the "exercise menu." The exercise menu may include, for example, jogging, paced running, build-up running, interval running, repetition running, and long slow distance (LSD).

Here, the jogging corresponds to, for example, running at an intensity of approximately 70% or less of the maximum heart rate. The paced running corresponds to, for example, running at a fixed pace that is designed for a target race. The build-up running corresponds to, for example, running with a gradual increase in pace. The interval running corresponds to, for example, a repetition of anaerobic sprinting and aerobic jogging. The repetition running corresponds to, for example, running where the runner runs a set distance at a near full effort pace, then rests and repeats the same running again. The LSD corresponds to, for example, running slower and longer independent of pace.

The setting unit 111 may use a data set equivalent to the VDOT-based calculation table proposed by Jack Daniels to set up the training program of the user. The VDOT corresponds to a pseudo-VO2max (maximum oxygen uptake) and an indicator of the current level of the running ability of the user, which can be inferred from, for example, a record under maximum effort in a recent race. From the VDOT, the completion time for a certain distance can be predicted. For example, if the VDOT is 30, the completion time for 10 km can be predicted as 1 hour 3 minutes 46 seconds, and the completion time for a half marathon as 2 hours 21 minutes 4 seconds. Hereinafter, the VDOT will be referred to as "running ability (running level (RL))." Here, the information needed to infer the running ability of the user may be obtained by running information obtained through the sensor device 301, input by the user, or other means. The setting unit 111 then estimates the anaerobic threshold (AT) value (an anaerobic work threshold) in accordance with the determined running ability and sets a guideline pace for the training menu.

The setting unit 111 also sets the training program on the basis of a predetermined training theory. The predetermined training theory may be on the basis of the concept of periodization, which is equivalent to the training phases proposed by Daniels above. The periodization is to divide the training period into several periods (phases) and to arrange the training menu in accordance with the running pace and distance for each period to adjust the peak condition and performance to the actual event such as a competition or game. The periodization may be configured by, but is not limited to, a preparation phase, a speed training phase, a running phase, a race simulation phase, and a recovery phase, for example. According to the concept of periodization, by implementing a training program that adjusts the type, amount, and intensity of training during each period, the growth rate of the running ability of the user follows a predetermined growth curve from the start of training to the actual performance period. The setting unit 111 may set a user-specific training program by adjusting the running pace and distance of the training menu to be implemented in each period on the basis of the current running ability (at the time of starting training) of the user as determined above.

In one embodiment of the present invention, the training program may divide a predetermined period of time from the start of training to the target competition into a predetermined number of chronological segments, and the training menu for each segment may be set in accordance with the running ability of the user on the basis of the concept of the periodization described above. The predetermined period may be 20 to 24 weeks, for example, but is not limited to this, and may be less than or equal to 20 weeks or greater than or equal to 24 weeks. Further, the training program may be configured such that training menus are arranged with one segment defined as one week (seven days), and each period of the periodization is configured by at least one segment (i.e., from one week to several weeks). That is, if the predetermined period is 20 weeks, the predetermined number may be set to 20 and one segment may be set to one week. However, the present invention is not limited to this, and one segment may be, for example, 5 days or even 10 days.

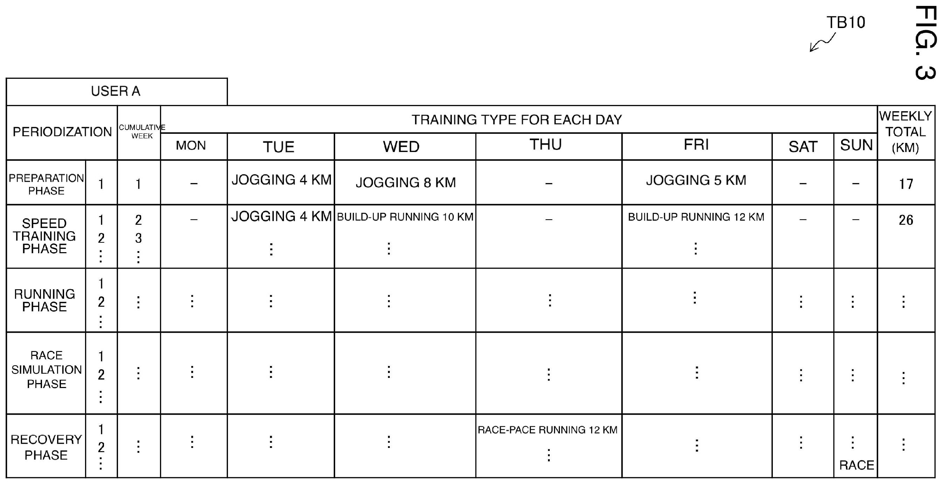

FIG. 3 shows an example of the set training program. FIG. 3 shows an example of a training program table TB10, which is set in accordance with the running ability of the user A, for example, and is stored in the database server 101. Note that FIG. 3 is a schematic and shows only a portion, and is not exact. In the example shown in FIG. 3, for the training program, training menus of jogging for 4 km, jogging for 8 km, and jogging for 5 km are arranged on Tuesday, Wednesday, and Friday, respectively, within one segment (one week) configuring a preparatory period. In addition, in the multiple segments (weeks) that configure the speed training phase, jogging for 4 km, build-up running for 10 km, and build-up running for 12 km are arranged on Tuesday, Wednesday, and Friday, respectively. In each period of the periodization, the training menu may be the same among the multiple segments that configure a period, or it may differ from one segment (week) to another. As described above, the training menu is configured in each segment until the last day of the recovery period (the final day of the predetermined period).

The display processing unit 115 of the server 100 displays information on the training menu to be implemented on that day to the user terminal 200 in accordance with the set training program. The user can check the training menu to be implemented on that day on the user terminal 200 via the training support application. Alternatively, the training menu to be implemented on that day may be notified in a pop-up display on the user terminal 200, or via email or existing communication applications.

The acquisition unit 112 acquires implementation information regarding whether the training menu set for the day of implementation is implemented by the user. The implementation information may correspond to information that can determine whether the training menu has been implemented by the user on the day of implementation, information entered by the user via the training support application, or running information acquired by the sensor device 301. The implementation information may be acquired by the acquisition unit 112, for example, when the user transmits to the server 100 that she/he has implemented the training via the training support application, or when the user terminal 200, in response to receiving running information acquired by the sensor device 301, transmits the running information to the server 100.

The determination unit 113 determines whether the training menu for the day of implementation has been implemented by the user. The determination unit 113 determines whether the user has implemented the preset training menu on the basis of the implementation information acquired by the acquisition unit 112. For example, the determination unit 113 determines whether the training menu of the day, e.g., "jogging for 4 km," has been implemented on the basis of the running information acquired by the sensor device 301 as implementation information. When the sensor device 301 is used, for example, a minimum value of the running speed or distance may be set in advance as a reference value, and on the basis of the reference value, it may be determined whether the training menu has been implemented. Alternatively, the determination unit 113 may determine whether the training menu has been implemented on the basis of information entered by the user via the training support application. For example, on the user terminal 200, a screen may be displayed to allow the user to select whether the "jogging for 4 km" as the training menu for the day has been implemented, or a screen may be displayed to allow the user to enter the details of the training implemented on that day, and the results of the selection and input by the user may be transmitted to the server 100.

Alternatively, the determination unit 113 may determine that the training menu on that day has not been implemented when implementation information fails to be acquired. The case where implementation information fails to be acquired may be, for example, when running information acquired by the sensor device 301 on the day fails to be present, or when implementation information via the training support application fails to be transmitted from the user terminal 200 to the server 100.

If the determination unit 113 determines that the training menu for the current day is not implemented, the update unit 114 updates training menus for subsequent days in accordance with predetermined conditions.

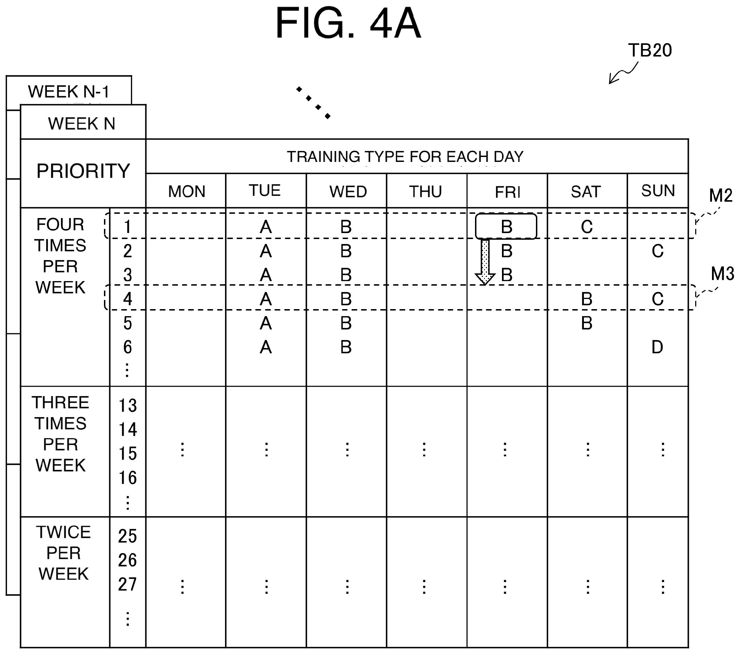

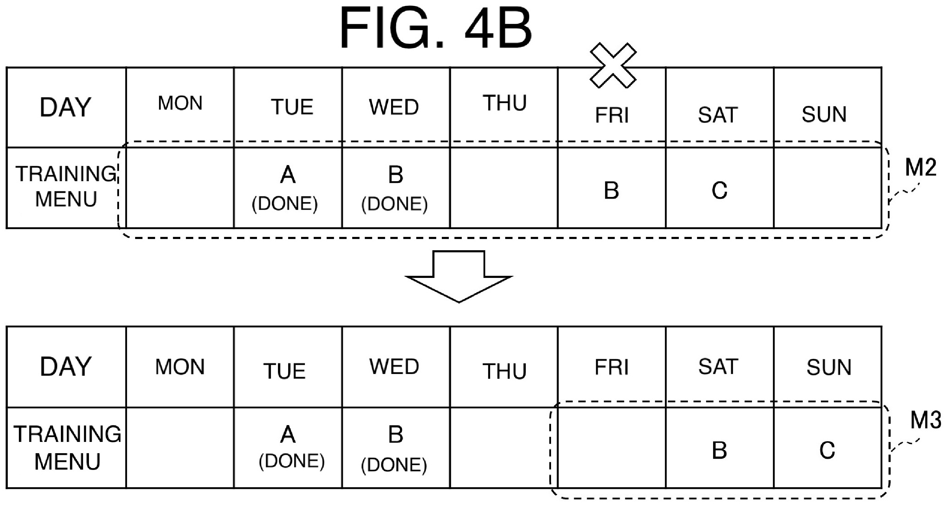

With reference to FIG. 4, the update of the training menu will be described. FIG. 4A shows an example of the first priority table stored in the database server 101. FIG. 4A is schematic and not exact. The first priority table TB20 corresponds to a table that indicates, for each of a predetermined number of chronologically divided segments of a predetermined period, multiple combinations of training menus and the dates of implementation of the training menus, as well as the priority order of the combinations. The predetermined period corresponds to the period from the beginning of the training to the target competition, as described above, during which the training program is implemented. Each of the predetermined number of chronologically divided segments of the predetermined period corresponds to one segment described above, which may be, for example, one week. The first priority table TB20 is set in advance for each segment on the basis of the periodization concept. For example, the first priority table TB20 may be stored for each segment (i.e., week), such as the first week of the preparation period, the second week or later of the speed training period, and the Mth week (M is a predetermined integer) or later of the running period, for example. FIG. 4A shows the first priority table TB20 for the Nth week (Nth segment).

The first priority table TB20 stores multiple combinations of training menus and the days of implementation of the training menus along with the priority order. In the example in FIG. 4A, the combination with the highest priority corresponds to the training menus "A," "B," and "C," which are implemented four times a week on Tuesday, Wednesday and Friday, and Saturday as days of implementation, respectively. The combination with the second highest priority corresponds to the training menus "A," "B," and "C," which are implemented four times a week with Tuesday, Wednesday and Friday, and Sunday as days of implementation, respectively. The first priority table TB20 may be used to set the training program by the setting unit 111. That is, the setting unit 111 may extract the combinations with the highest priority from the first priority table TB20 for each segment, respectively, and set them as the training program at the start of training after corrections in accordance with the running ability of the user. When the training program is configured by three times of training per week, in the first priority table TB20, the highest priority of the combinations by three times of training per week (in the case of FIG. 4A, the combination with the thirteenth-highest priority) may be extracted for each segment and the training program may be set.

FIG. 4B is used for further description. Now, consider the case where a user that trains using the combination M2 of training menus has not yet implemented the training menu "B" that should be performed on Friday of the Nth week. At this time, the update unit 114, with reference to the first priority table TB20 shown in FIG. 4A, sequentially checks the combinations of the training menus located below a combination M2 of the training menu, and updates the training menus for subsequent days to a combination of training menus in which a training menu on Friday is first empty. In the example of FIG. 4A, among the combinations of the training menus located below the combination M2, the combination M3 is the first combination having a blank training menu on Friday. Therefore, in the Nth week, as shown in FIG. 4B, the training menu after Saturday is updated to the training menu of the combination M3.

In the first priority table TB20, the priorities among training menus within a training period are set in accordance with the concept of the periodization. Further, the priority is set with the intention of limiting the training menu so that it is not implemented on consecutive days to protect the user from being injured. This allows the training program to be updated to cover unimplemented training menus after the day of no training according to one embodiment of the present invention.

As described above, according to one embodiment of the present invention, even if the user is unable to implement the training menu scheduled for the day, training menus scheduled for one or more subsequent days are updated to cover the unimplemented training menu. This limits the reduction of the running ability and motivation of the user and maintains efficient training.

The display processing unit 115 displays the information on the updated training menu on the user terminal 200. The information on the training menu may be any information capable of notifying the user, for example, that the training "B" is to be implemented on Friday, and time points for displaying the information are not limited. For example, the fact that the training menu "B" is to be implemented on Friday may be notified at a predetermined time on Thursday night, or it may be displayed at the time point when the user runs the training support application on Friday. Alternatively, the user terminal 200 may notify the user that the training menu has been changed after Friday.

### Control Flowchart of Server

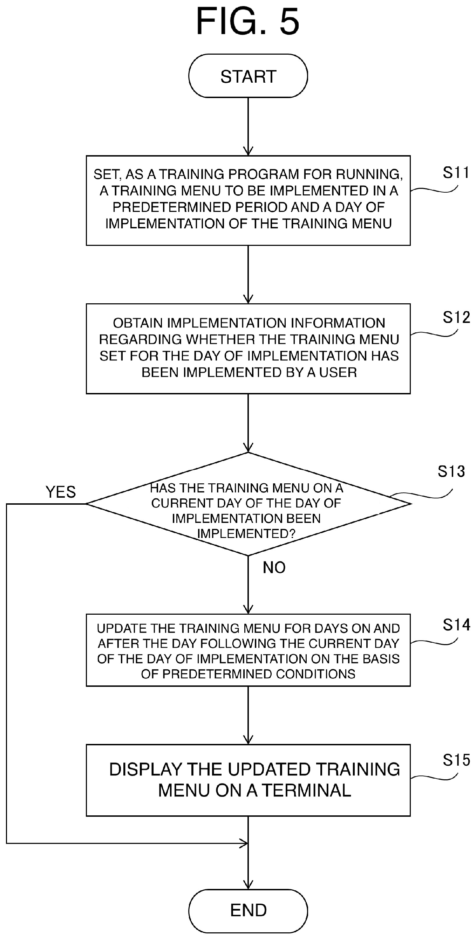

With reference to the flowchart in FIG. 5, the control method of the server 100 described above will be described. The function of the prediction unit 116 will be described below. As described above, the setting unit 111 of the server 100 sets a training menu to be implemented during a predetermined period as a training program related to running, and the day of implementation of the training menu (step S11). The acquisition unit 112 acquires implementation information regarding whether the training menu set for the day of implementation is implemented by the user (step S12). The determination unit 113 determines whether the training menu for the day of implementation has been implemented (step S13). If the determination unit 113 determines that the training menu for the day of implementation has been implemented (YES in step S13), the process is terminated. If the determination unit 113 determines that the training menu for the day of implementation has not been implemented (NO in step S13), the update unit 114 updates the training menus for subsequent days in accordance with the predetermined conditions (step S14). The display processing unit 115 displays the updated training menu on the terminal (step S15).

As described above, according to one embodiment of the present invention, if the user has not implemented the training in accordance with the training program, the training program on the next day or later may be updated to cover the unimplemented training menu. In addition, the user is notified that the training program has been updated, which helps maintain the motivation of the user to continue the training.

### Other Embodiments

According to one embodiment of the present invention, the growth of the running ability of the user may be predicted in accordance with the implementation status of the training program. That is, the prediction unit 116 predicts the running ability of the user at the end of the predetermined period in accordance with the implementation status of the training menu in the predetermined period on the basis of the implementation information. For example, the growth prediction of the running ability may be updated when the intensity at which the training menu is implemented is changed by the user. Further, in the above description, a form has been described in which the training menus for subsequent days are updated when the training menu scheduled for the day of implementation has not been implemented by the user. According to one embodiment of the present invention, if a period during which the set training menus remain unimplemented extends over one segment, updating of the training menus differs from updating performed when the training menu scheduled for the day of implementation has not been implemented. Hereinafter, these forms will be described.

### Case where Intensity of Training is Changed

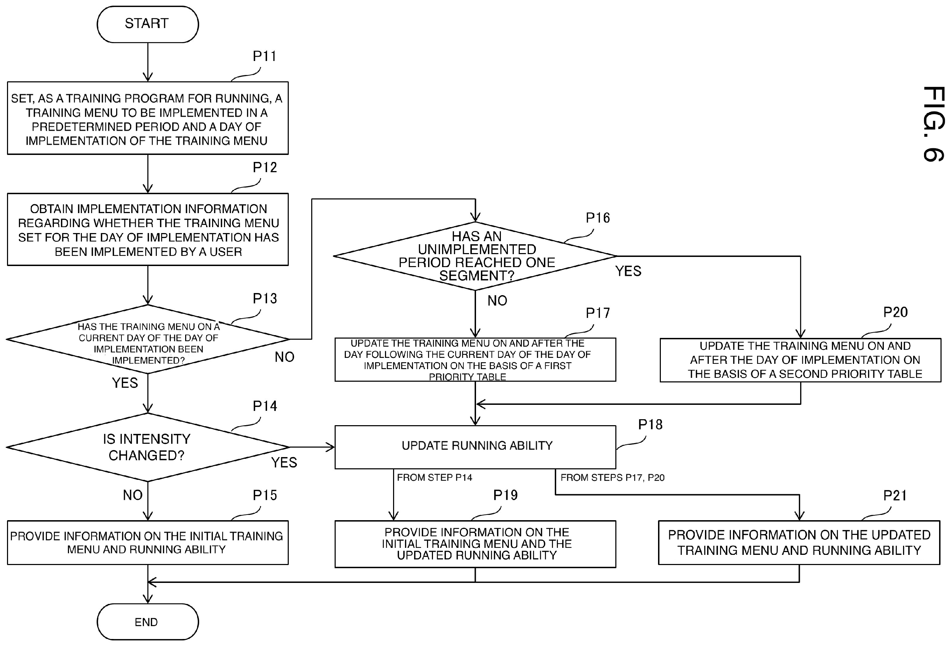

FIG. 6 shows a control flowchart illustrating a control of the server 100 according to an embodiment of the present invention. First, the server 100 determines whether the user has implemented the training menu for the day of implementation through steps P11 to P13. Since steps P11 to P13 are the same as steps S11 to S13 in FIG. 5, the description thereof will be omitted. If the determination unit 113 determines in step P13 that the user has implemented the training menu for the day of implementation (YES in step P13), the determination unit 113 determines whether the intensity of the training menu is changed on the basis of the implementation information (step P14). The intensity of the training menu corresponds to the level of load when the training menu is implemented. For example, a table may be stored in the database server 101, in which a training menu with a stronger load and a training menu with a weaker load are associated for each training menu.

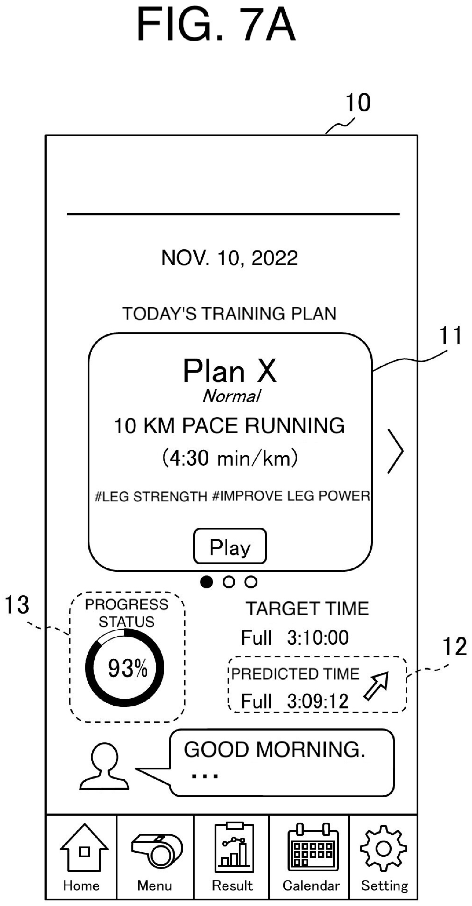

With respect to the change in intensity, the display processing unit 115 displays, on the user terminal 200 on the day of implementation, a selection screen that allows the user to select a load level for the training menu set for that day. FIGS. 7A to 7C show an example of a selection screen. FIGS. 7A to 7C show an example and are not limited to the manner in which it is displayed or the wording. The selection screen may display a selection menu in accordance with the level of load and information on the running ability at the end of the predetermined period of training that is predicted when the selected level of load is implemented. The "information on the running ability" may be numerical values indicating the running ability itself, or may be a growth rate in the running ability from the start to the end of training. Alternatively, it may be a predicted time needed to complete a target competition, or a degree of reduction in the predicted time in comparison to that at the start of training.

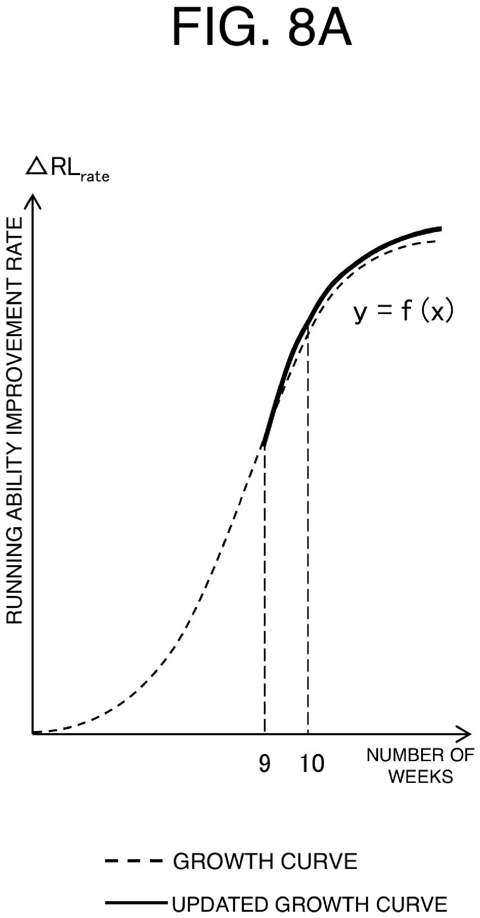

The prediction of the running ability by the prediction unit 116 will be described here. According to the concept of the periodization as described above, by implementing the set training program, the growth rate (rate of improvement) of the running ability of the user draws a predetermined growth curve over the period from the start of training to the actual performance. FIGS. 8A to 8C each show an example of the growth rate of the running ability (growth curve). The curve shown by the dashed line in each of FIGS. 8A to 8C (y = f(x)) shows an example of the growth curve. The prediction unit 116 may predict the running ability at the end of the predetermined period on the basis of the rate of improvement in the running ability (growth curve) acquired by the implementation of the training program by the user over the predetermined period. That is, the prediction unit 116 may predict the training ability at the end of the training program, assuming that the training ability will grow along the growth curve if the user implements the training as in the set training program. FIGS. 8A to 8C will be described below.

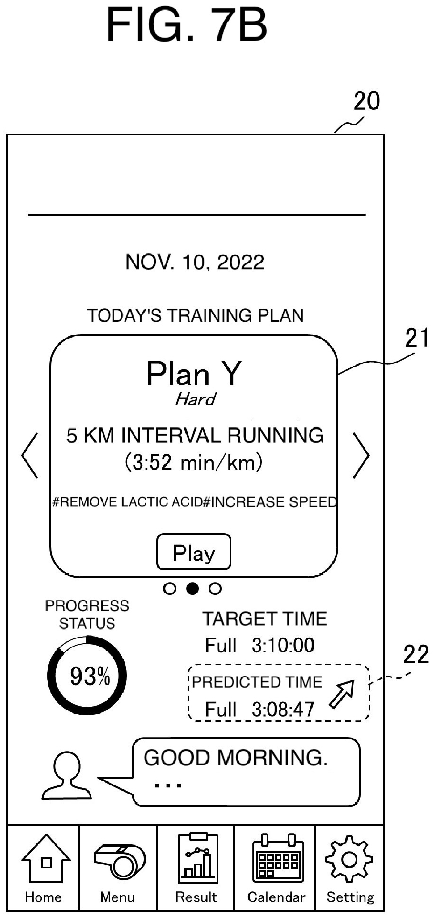

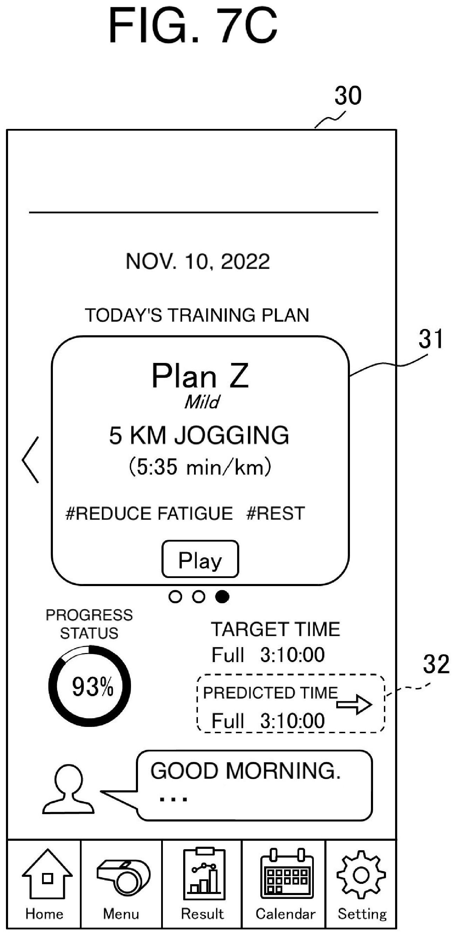

Returning to FIG. 7, the selection of the level of load continues to be described. The screen 10 in FIG. 7A shows a training menu 11 with a reference intensity set by the setting unit 111, which is a "paced running for 10 km." In addition, the screen 10 displays, as the information on the running ability predicted when the training menu 11 is implemented, for example, a predicted time 12 needed to complete the target competition. The screen 20 in FIG. 7B shows "interval running for 5 km" as a training menu 21 with a higher load than the training menu with the reference intensity. Since it corresponds to a training menu with a higher intensity, on the screen 20, a predicted time 22 is predicted to be earlier than the predicted time 12 for the reference intensity. The screen 30 in FIG. 7C shows "jogging for 5 km" as a training menu 31 with a lower load than the training menu with the reference intensity. Since it corresponds to a training menu with a lower intensity, on the screen 30, a predicted time 32 is predicted to be slower than the predicted time 12 for the reference intensity. The update of the predicted time will be described below. As shown in FIGS. 7A to 7C, arrows may be displayed to indicate an increase, decrease, or maintenance of the predicted time so that the user is aware that the predicted time is improving. The user may select his/her own training menu from screens 10 to 30, and the selected information may be transmitted from the user terminal 200 to the server 100.

If the user selects the training menu with a reference intensity (NO in step P14), information on the training menu initially set and the running ability is provided to the user (step P15). If the user selects a training menu with an intensity different from the reference intensity (higher load or lower load) (YES in step P14), the prediction unit 116 updates the running ability predicted for the user (step P18). The updated information may be stored in the database server 101.

With reference to FIG. 8A, the updating of the running ability by changing the intensity will be described. The prediction unit 116 updates the running ability at the end of the predetermined period in accordance with the rate of improvement in the running ability corrected by the weight coefficient for the level of load selected by the user. In the graph in FIG. 8A, the dashed line represents an initial growth rate (growth curve) of the running ability, and corresponds to, for example, a growth curve obtained when the predetermined period is set to 20 weeks and the training is implemented four times per week at a reference intensity. Assume that a training menu with a high intensity has been selected for a certain training menu in the 10th week. In this case, the prediction unit 116 may correct the growth rate from the 9th week to the 10th week using a predetermined constant corresponding to the high load and a coefficient set in accordance with the age of the user. As a result, the growth rate after the 10th week is shifted from the initial growth rate by an amount corresponding to the corrected growth rate from the 9th week to the 10th week, thereby forming a curve indicated by a solid line in FIG. 8A. The prediction unit 116 predicts the running ability at the end of the training on the basis of the corrected growth rate. When a training menu with a lower load is selected, it may be corrected in the same manner using a predetermined constant corresponding to the lower load and a coefficient related to age.

After step P18, the system proceeds to step P19, where the user is provided with information on the initially set training menu and updated running ability.

As described above, the intensity of the training menu may be selectable by the user. This allows the user to select a training menu that can be implemented in accordance with the physical condition and the availability of time of the user, thereby facilitating continued training. In addition, information on the training ability expected to be acquired for each intensity of training is provided to the user, which is expected to have the effect of maintaining the motivation of the user.

### Case where one or more Training Menus are Unimplemented

Next, with reference to the flowchart in FIG. 6, the updating of the training program when a period during which the set training menu is unimplemented extends over one segment will be described. If the determination unit 113 determines in step P13 that the training menu for the day of implementation has not been implemented yet (NO in step P13), the process proceeds to step P16, where the determination unit 113 determines whether the unimplemented period has reached one segment. That is, here, the determination unit 113 may determine whether the days on which the training has not been implemented have continued for one week. If the determination unit 113 determines that a period during which the training is not implemented has reached one segment (one week) (YES in step P16), the update unit 114 updates the training menu for the following day and thereafter on the basis of the second priority table (step P20). The second priority table TB30 corresponds to a table indicating the priority order of the segments to be deleted among multiple segments and may be stored in the database server 101.

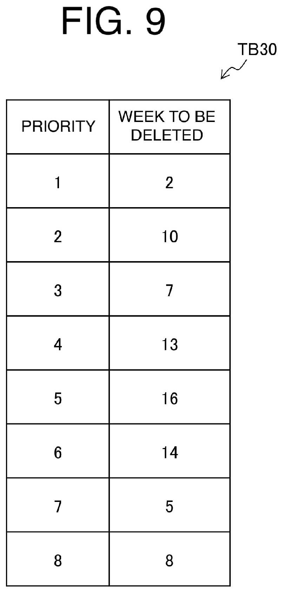

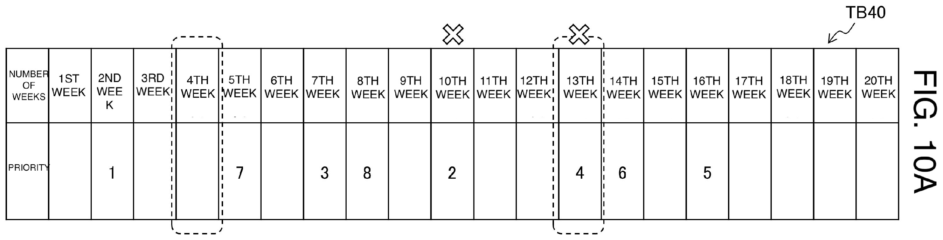

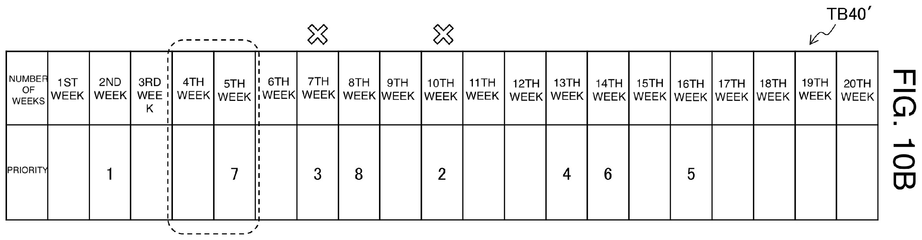

The update unit 114 updates the training menu by deleting, on the basis of the second priority table, training menus of segments with higher priorities to be deleted among segments from and after the segment that has not been implemented. FIG. 9 shows an example of the second priority table TB30. FIGS. 10A and 10B show schematic diagrams to illustrate updating the training program using the second priority table TB30. In the example of FIGS. 10A and 10B, a training program is set for a period of 20 weeks, and priorities of weeks to be deleted are associated with the respective weeks and shown. Assume that in the Table TB40 in FIG. 10A, the user has not implemented the training for the fourth week yet. At this time, the update unit 114 deletes a training menu set for the 10th week with the highest priority to be deleted among weeks from and after the 4th week. The training menus set for the 4th week to the 9th week before the updating are then shifted to be training menus for the 5th week to the 10th week. Further, in the table TB40, for example, if the user has not implemented training in the 13th week, the update unit 114 deletes a training menu set for the 13th week as a week with the highest priority to be deleted among weeks from and after the 13th week. In the same manner, assume that in the Table TB40' in FIG. 10B, the user has not implemented training for two weeks, the 4th week and the 5th week. In this case, the update unit 114 deletes, among weeks from and after the 4th week, a training menu set for the 10th week as a week the highest priority to be deleted, and deletes, among weeks from and after the 5th week, a training menu set for the 7th week as a week with the second-highest priority to be deleted. The training menus set for the 4th, 5th, 6th, 8th, and 9th weeks before the updating are then set as training menus for the 6th week to the 10th week, respectively.

Returning to the flowchart in FIG. 6, after step P20, the prediction unit 116 updates the predicted running ability (step P18). When the training menu has not been performed by the user over one segment, the prediction unit 116 predicts the running ability at the end of the predetermined period by subtracting the rate of improvement of the running ability obtained when the training menu set for the one segment has been implemented from the rate of improvement of the running ability obtained by implementing the training program over the predetermined period.

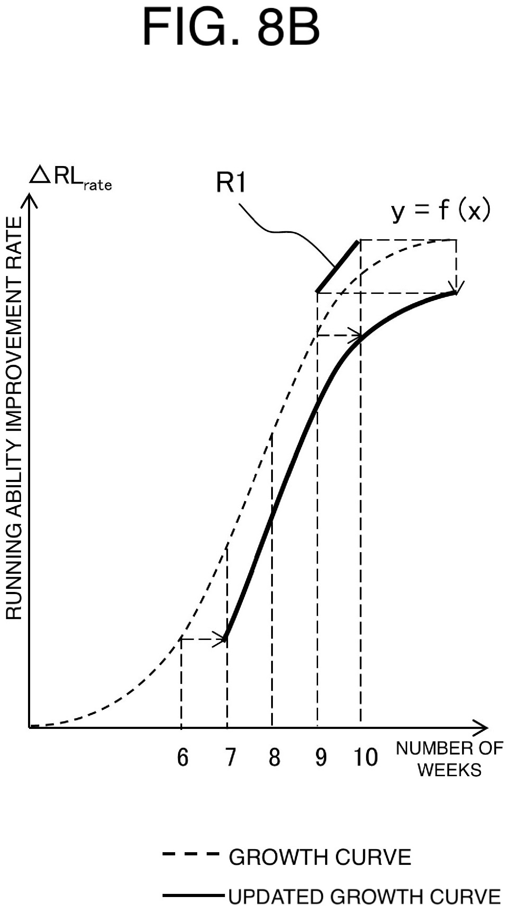

This will be illustrated with reference to FIG. 8B. In FIG. 8B, in the same manner as in FIG. 8A, a dashed-line curve represents a growth curve obtained when the predetermined period is set to 20 weeks and the training is implemented four times per week with a reference intensity. Now assume that the training for the 7th week has not been implemented and the training menu set for the 10th week is deleted on the basis of the second priority table TB30. In this case, the prediction unit 116 updates the growth rate by deleting the growth rate that has been expected to be earned in the 10th week. As a result, the growth rate after the 7th week is reduced from the initial growth rate by an amount of the deleted growth rate, thereby forming a curve indicated by a solid line in FIG. 8B.

After step P18, the system proceeds to step P21, where the user is provided with information on the updated training menu and the running ability.

As described above, according to one embodiment of the present invention, even if the period of time during which training has not been implemented is extended to some extent, the training program is updated in consideration of the period of time during which the training has not been implemented. Accordingly, a user-friendly and efficient training program is provided, facilitating the continued training by the user. In addition, since the predicted value of the running ability is updated in consideration of the period of time during which the training has not been implemented, it does not cause the user to feel discouraged and contributes to maintaining the motivation.

### Case where the Number of Trainings is Reduced

If the period of time during which the training has not been implemented is less than one week (NO in step P16), the update unit 114 updates the training menu for the following day and thereafter on the basis of the first priority table TB20 as described above (step P17).

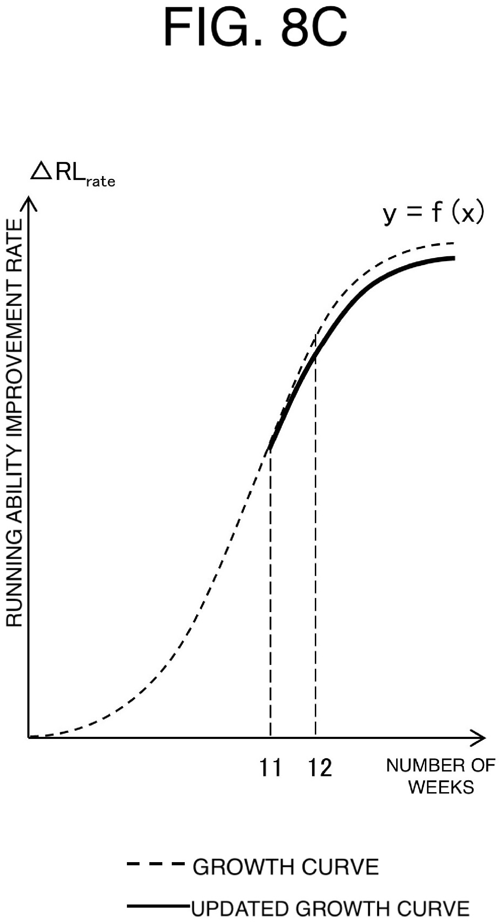

After step P17, the prediction unit 116 updates the running ability in accordance with the updated contents of the training menu (step P18). For example, if the number of trainings in one segment is reduced due to the updated training menu, the running ability may be updated. This will be illustrated with reference to FIG. 8C. In FIG. 8B, in the same manner as in FIG. 8A, a dashed-line curve represents a growth curve obtained when the predetermined period is set to 20 weeks and the training is implemented four times per week with a reference intensity. Now assume that the number of trainings for the week is reduced from the initial four to three because the training has not been implemented on Thursday of the 12th week. In this case, the prediction unit 116 may correct the growth rate from the 11th week to the 12th week with a preset weighting in accordance with the number of trainings for each segment. As a result, the growth rate after the 12th week is shifted from the initial growth rate by an amount corresponding to the corrected growth rate from the 11th week to the 12th week, thereby forming a curve indicated by a solid line in FIG. 10B.

After step P18, the system proceeds to step P21, where the user may be provided with information on the updated training menu and the running ability. If the number of trainings within one segment remains unchanged in step P17, the running ability need not be updated.

Although the present invention has been described on the basis of the drawings and embodiments, it should be noted that one skilled in the art can easily make various changes and modifications on the basis of the present disclosure. Accordingly, it should be noted that these changes and modifications are included in the scope of the present invention. For example, functions included in respective means, respective steps, for example, may be rearranged if no logical inconsistency arises, and a plurality of means or steps may be combined into one or divided into multiple parts. In addition, configurations shown in the above embodiments may be appropriately combined. For example, the components described as provided by the server 100 may be distributed and embodied by multiple servers. The processes described as functions of the server 100 may also be performed by the user terminal 200 and sensor devices 301, 302. Conversely, the process assumed to be performed by the user terminal 200 may be performed by the server 100 and sensor devices 301, 302.

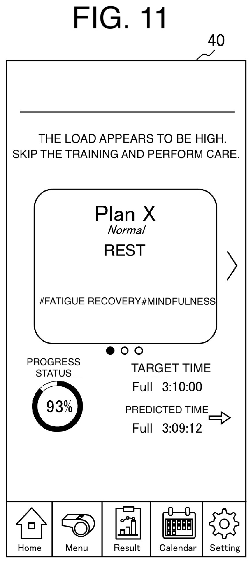

For example, in the above description, a case has been described in which a user is allowed to select intensity of the training menu. In addition to this, according to one embodiment of the present invention, the user may be notified of the recommendation to rest. The screen 40 in FIG. 11 may show an example of a notification screen displayed on the user terminal 200 that prompts the user to rest. For example, a deviation from the usual running form of the user may be determined by the determination unit 113 on the basis of, for example, the landing impact included in the running information of the sensor device 301, and if the deviation exceeds a predetermined threshold value, it may be determined that the rest is needed. The screen 40 may then be displayed by the display processing unit 115. At this time, the prediction unit 116 may update the expected running ability at the end of the training program in response to the rest (i.e., not implementing the training), convert it to a completion time, and display it on the screen 40. This provides a training program capable of maintaining the motivation of the user as well as limiting the injuries of the user.

The second priority table TB30 may also be used when the training program is set up for weeks other than the 20th week. For example, when a training program for a full marathon is set up, a training program may be set up with a reference training period of 20 weeks, a minimum of 12 weeks, and a maximum of 24 weeks. If the training period is shorter than the standard 20 weeks, a training program may be configured by referring to the second priority table TB30 and removing training menus for weeks with higher deletion priorities. For example, if the training period is 19 weeks, a training program excluding a training menu for the 2nd week with the highest priority may be configured.

Although not shown in FIG. 9, if the training period is longer than the standard 20 weeks, the second priority table TB30 may store a number of weeks in which training menus to be added are arranged, associated with positions at which the number of weeks is to be inserted. For example, if the training period is 21 weeks, a training program may be set up in which a training menu assigned to the 7th week is added before the 8th week. If the training period is 22 weeks, a training program may be set up in which the training menu assigned to the 7th week is added before the 8th week, and then the training menu assigned to the 16th week is added before the 15th week.

In addition, as shown in FIG. 7, the user terminal 200 displays the progress status up to the current day in the training program set for the user. For example, as the progress status, a progress rate of the training program may be presented to the user by using a progress ring 13 displayed on the screen 10 in FIG. 7A, for example. The update unit 114 may also update the rate of progress in accordance with the implementation of the training program by the user. The update of the progress rate may be performed as follows, for example, but not limited to this.

### Progress Rate per Week

If the training program is evenly assigned to each week, e.g., a training program with 20 weeks is implemented as initially set, the rate of progress may increase by 5% in each week. In addition, in accordance with the shape of the predetermined growth curve described above, a value of a function f(x) of the growth curve may be used such that, for example, if a progress rate from the 14th week to the 15th week is ΔRL = f(15) - f(14) = 7% and a training menu for the 14th week is implemented as set, a progress rate of 7% may be applied.

### Progress Rate per Training Menu

The progress rate is evenly allocated to each training menu, and if a given week has a progress value of 5% and three training sessions have been implemented, the progress rate may be of 5 × 0.75 = 3.75%. Additionally, in accordance with the weight of the training menu for each week, if a given week has a progress value of 5% and training with a weight of 10% has not been implemented, the progress rate may be of 5 × 0.9 = 4.5%.

### Degree of Completion of the Training Menu

Considering whether the training menu has been implemented, the level of achievement for each training menu is evaluated with 1 for implemented and 0 for unimplemented. Additionally, the achievement level may be computed from constants in accordance with the following: "the training menu has been implemented," "60% of the distance specified in the training menu has been covered," or "at least 60% of the distance specified in the training menu has been covered and the average pace has been faster than the specified pace plus 10 seconds per km (however, if the specified pace has a range, the lower limit (slower side) of the pace plus 10 seconds per km)." For example, in a certain week, if the current progress rate is 7% and the achievement level of training weighted at 30% is 90%, then 7 × 0.3 × 0.9 = 1.89% may be treated as the progress rate.

The functional units of the server 100 or the information processing device 100 may be embodied by logic circuits (hardware) or dedicated circuits formed in integrated circuits (IC) chips and large scale integration (LSI), for example, or by software using a central processing unit (CPU). Each functional unit may be embodied by one or more integrated circuits, and the functions of multiple functional units may be embodied by a single integrated circuit.

The programs of each embodiment of the present disclosure may be provided as stored in a storage medium readable by an information processing device. The storage medium is directed to a "non-transitory tangible medium" capable of storing programs. The programs may include, for example, software programs and information processor programs. When the functional units of the information processing device 100 are embodied by software, the information processing device 100 functions as the setting unit 111, acquisition unit 112, determination unit 113, update unit 114, display processing unit 115, and prediction unit 116 by causing a processor to execute a program loaded into the memory.

The storage medium may include, as appropriate, one or more semiconductor-based or other integrated circuits (ICs) (for example, a field programmable gate array (FPGA) or an application specific integrated circuit (ASIC)), a hard disk drive (HDD), a hybrid hard drive (HHD), an optical disk, an optical disk drive (ODD), a magneto-optical disk, a magneto-optical drive, a floppy (registered trademark) diskette, a floppy (registered trademark) disk drive (FDD), a magnetic tape, a solid-state drive (SSD), a RAM drive, a secure digital card or drive, any other suitable storage medium, or any suitable combination of two or more thereof. The storage medium may be volatile, nonvolatile, or a combination of volatile and nonvolatile, as appropriate.

The program of this disclosure may be provided to the information processing device 100 via any transmission medium (e.g., communication network, and broadcast wave) capable of transmitting the program.

The embodiments of the present disclosure can also be embodied in the form of data signals embedded in a carrier wave, in which the program is embodied by electronic transmission. The program according to the present disclosure may be implemented using, for example, scripting languages such as JavaScript (registered trademark) and Python, or programming languages such as C, Go, Swift, Kotlin, and Java (registered trademark).

It is understood by those skilled in the art that the embodiments described above correspond to specific examples of the following aspects.
[1] The information processing according to the present disclosure is directed to an information processing device that provides a user with a training program related to running. The information processing device includes: a setting unit configured to set, as the training program, a training menu to be implemented in a predetermined period and a day of implementation of the training menu; an acquisition unit configured to acquire implementation information regarding whether the training menu set for the day of implementation has been implemented by the user; a determination unit configured to determine whether the training menu for a current day of the day of implementation has been implemented; an update unit configured to update, on the basis of predetermined conditions, training menus for days following the current day of the day of implementation if the determination unit determines that the training menu for the current day of the day of implementation has not been implemented; and a display processing unit configured to cause a terminal to display the updated training menus.
[2] In the information processing device of [1] above further includes a prediction unit configured to predict running ability of the user at an end of the predetermined period in accordance with an implementation status of the training menu in the predetermined period on the basis of the implementation information. The prediction unit predicts the running ability of the user at the end of the predetermined period in a case where a load level of the set training menu is changed, and the display processing unit causes the terminal to display, on a current day of the day of implementation, a selection screen for allowing the user to select the load level for the training menu set for the current day, the selection screen including information on the running ability predicted at the end of the predetermined period in accordance with the load level.
[3] In the information processing device according to [1] or [2] above, the setting unit may set the training program on the basis of a first priority table stored in a predetermined storage, the first priority table indicating a plurality of types of combinations for each segment obtained by dividing the predetermined period into a predetermined number in a time series, each combination including the training menu and a day of implementation of the training menu, and priorities of the combinations, and if the training menu for the current day of the day of implementation has not been implemented, the update unit may update training menus for days following the current day of the day of implementation on the basis of the first priority table to training menus on the basis of a combination with a higher priority among combinations that include the training menu that has not been implemented on or after the current day of the day of implementation.
[4] In the information processing device according to [3] above, if the training menu set for one segment remains unimplemented over the one segment, the update unit may update the training menu by deleting training menus of a segment with a higher priority among segments to be deleted, on or after the unimplemented segment, on the basis of a second priority table stored in the predetermined storage, the second priority table indicating priorities of segments to be deleted among the plurality of segments.
[5] In the information processing device according to [2] above, the setting unit may set the training program on the basis of a predetermined training theory, and the prediction unit may predict the running ability of the user at the end of the predetermined period on the basis of a rate of improvement in running ability obtained by the user implementing the training program over the predetermined period.
[6] In the information processing device according to [2] above, the prediction unit may update the running ability at the end of the predetermined period in accordance with a rate of improvement of the running ability corrected by a weight factor in accordance with the load level selected by the user.
[7] In the information processing device according to [2] above, if the training menu remains unimplemented by the user over one segment, the prediction unit may update the running ability at the end of the predetermined period by subtracting a rate of improvement of running ability obtained when a training menu set for the one segment is implemented from a rate of improvement of running ability obtained by implementing the training program over the predetermined period.
[8] In the information processing device according to [2] above, the display processing unit may cause the selection screen to display predicted time to complete a predetermined distance as information on the running ability at the end of the predetermined period.
[9] In the information processing device according to [2] above, the display processing unit may cause the selection screen to display a progress status up to the current day in the training program set by the user.
[10] In the information processing device according to any one of [1] to [9] above, the acquisition unit may acquire information on a physical load applied to the user from a sensor worn by the user, and the update unit may update subsequent training menus to training menus with a lower load in response to the physical load applied to the user satisfying a predetermined condition.
[11] A method for controlling an information processing device that provides a user with a training program related to running according to the present disclosure causes the information processing device to perform the steps of: setting, as the training program, a training menu to be implemented in a predetermined period and a day of implementation of the training menu; acquiring implementation information regarding whether the training menu set for the day of implementation has been implemented by the user; determining whether the training menu for a current day of the day of implementation has been implemented; updating, on the basis of predetermined conditions, training menus for days following the current day of the day of implementation if it is determined that the training menu for the current day of the day of implementation has not been implemented; and causing a terminal to display the updated training menu.
[12] A program for controlling an information processing device that provides a user with a training program related to running according to the present disclosure causes the information processing device to embody the functions of: setting, as the training program, a training menu to be implemented in a predetermined period and a day of implementation of the training menu; acquiring implementation information regarding whether the training menu set for the day of implementation has been implemented by the user; determining whether the training menu for a current day of the day of implementation has been implemented; updating, on the basis of predetermined conditions, training menus for days following the current day of the day of implementation if it is determined that the training menu for the current day of the day of implementation has not been implemented; and causing a terminal to display the updated training menu.

### Description of Reference Numerals

100: Server (Information Processing Device)
110: Controller
111: Setting Unit
112: Acquisition Unit
113: Determination Unit
114: Update Unit
115: Display Processing Unit
116: Prediction Unit
120: Communication Unit
130: Display
170: Storage
101: Database Server
200: User Terminal (Communication Terminal)
210: Controller
211: Sensor Data Acquisition Unit
220: Communication Unit
230: Display
240: Input/Output Unit
270: Storage
301: Sensor Device (Motion Sensor)
302: Sensor Device (Vital Sensor)
310: Controller
320: Communication Unit
330: Sensor
340: Operation Unit
350: Storage
500: Network
600: Training Support System

## Claims

1. An information processing device that provides a user with a training program related to running, comprising:
a setting unit configured to set, as the training program, a training menu to be implemented in a predetermined period and a day of implementation of the training menu;
an acquisition unit configured to acquire implementation information regarding whether the training menu set for the day of implementation has been implemented by the user;
a determination unit configured to determine whether the training menu for a current day of the day of implementation has been implemented;
an update unit configured to update, on the basis of predetermined conditions, training menus for days following the current day of the day of implementation if the determination unit determines that the training menu for the current day of the day of implementation has not been implemented; and
a display processing unit configured to cause a terminal to display the updated training menus.

2. The information processing device according to claim 1, further comprising:
a prediction unit configured to predict running ability of the user at an end of the predetermined period in accordance with an implementation status of the training menu in the predetermined period on the basis of the implementation information, wherein
the prediction unit predicts the running ability of the user at the end of the predetermined period in a case where a load level of the set training menu is changed, and
the display processing unit causes the terminal to display, on a current day of the day of implementation, a selection screen for allowing the user to select the load level for the training menu set for the current day, the selection screen including information on the running ability predicted at the end of the predetermined period in accordance with the load level.

3. The information processing device according to claim 1, wherein
the setting unit sets the training program on the basis of a first priority table stored in a predetermined storage, the first priority table indicating a plurality of types of combinations for each segment obtained by dividing the predetermined period into a predetermined number in a time series, each combination including the training menu and a day of implementation of the training menu, and priorities of the combinations, and
if the training menu for the current day of the day of implementation has not been implemented, the update unit updates training menus for days following the current day of the day of implementation on the basis of the first priority table to training menus on the basis of a combination with a higher priority among combinations that include the training menu that has not been implemented on or after the current day of the day of implementation.

4. The information processing device according to claim 3, wherein if the training menu set for one segment remains unimplemented over the one segment, the update unit updates the training menu by deleting training menus of a segment with a higher priority among segments to be deleted, on or after the unimplemented segment, on the basis of a second priority table stored in the predetermined storage, the second priority table indicating priorities of segments to be deleted among the plurality of segments.

5. The information processing device according to claim 2, wherein
the setting unit sets the training program on the basis of a predetermined training theory, and
the prediction unit predicts the running ability of the user at the end of the predetermined period on the basis of a rate of improvement in running ability obtained by the user implementing the training program over the predetermined period.

6. The information processing device according to claim 2, wherein the prediction unit updates the running ability at the end of the predetermined period in accordance with a rate of improvement of the running ability corrected by a weight factor in accordance with the load level selected by the user.

7. The information processing device according to claim 2, wherein if the training menu remains unimplemented by the user over one segment, the prediction unit updates the running ability at the end of the predetermined period by subtracting a rate of improvement of running ability obtained when a training menu set for the one segment is implemented from a rate of improvement of running ability obtained by implementing the training program over the predetermined period.

8. The information processing device according to claim 2, wherein the display processing unit causes the selection screen to display predicted time to complete a predetermined distance as information on the running ability at the end of the predetermined period.

9. The information processing device according to claim 2, wherein the display processing unit causes the selection screen to display a progress status up to the current day in the training program set by the user.

10. The information processing device according to claim 1, wherein
the acquisition unit acquires information on a physical load applied to the user from a sensor worn by the user, and
the update unit updates subsequent training menus to training menus with a lower load in response to the physical load applied to the user satisfying a predetermined condition.

11. A method for controlling an information processing device that provides a user with a training program related to running, the method causing the information processing device to perform the steps of:
setting, as the training program, a training menu to be implemented in a predetermined period and a day of implementation of the training menu;
acquiring implementation information regarding whether the training menu set for the day of implementation has been implemented by the user;
determining whether the training menu for a current day of the day of implementation has been implemented;
updating, on the basis of predetermined conditions, training menus for days following the current day of the day of implementation if it is determined that the training menu for the current day of the day of implementation has not been implemented; and
causing a terminal to display the updated training menu.

12. A program for controlling an information processing device that provides a user with a training program related to running, the program causing the information processing device to embody the functions of:
setting, as the training program, a training menu to be implemented in a predetermined period and a day of implementation of the training menu;
acquiring implementation information regarding whether the training menu set for the day of implementation has been implemented by the user;
determining whether the training menu for a current day of the day of implementation has been implemented;
updating, on the basis of predetermined conditions, training menus for days following the current day of the day of implementation if it is determined that the training menu for the current day of the day of implementation has not been implemented; and
causing a terminal to display the updated training menu.
